# EUROPEAN PATENT SPECIFICATION

(11) **EP 4 525 978 B1**
(45) Date of publication and mention of the grant of the patent: **06.05.2026**
(21) Application number: 23722522.2
(22) Date of filing: 26.04.2023
(51) Int. Cl.: A61N 1/372, H02J 50/90, A61N 1/378, H02J 50/10

(54) **SYSTEM COMPRISING AN IMPLANTABLE MEDICAL DEVICE AND AN EXTERNAL DEVICE**
SYSTEM MIT EINER IMPLANTIERBAREN MEDIZINISCHEN VORRICHTUNG UND EINER EXTERNEN VORRICHTUNG
SYSTÈME COMPRENANT UN DISPOSITIF MÉDICAL IMPLANTABLE ET UN DISPOSITIF EXTERNE

(30) Priority: 18.05.2022 US 202263343296 P; 15.06.2022 EP 22179078
(43) Date of publication of application: 26.03.2025
(73) Proprietor: BIOTRONIK SE & Co. KG, 12359 Berlin (DE)
(72) Inventor: TAFF, Brian M., Portland, Oregon 97217 (US); MCMILLAN, Brad, Lake Oswego, Oregon 97035 (US); MILLER, David, Lake Oswego, Oregon 97035 (US)
(74) Representative: Biotronik Corporate Services SE
(86) International application number: PCT/EP2023/060908
(87) International publication number: WO 2023/222347

(56) References cited:
- US-A1- 2011 184 491
- US-A1- 2020 178 802
- US-A1- 2020 405 149
- US-B1- 6 510 345

## Description

The invention is directed to a system comprising an implantable medical device (IMD) and an external device configured to communicate with the implantable medical device as well as to a respective external device, and an operation method of such external device. The invention is further directed to respective computer program product and a respective computer readable data carrier.

Usually, a medical device, in particular, an implantable medical device, is configured to monitor the health status of a patient and/or to deliver a therapy signal to the patient.

Active and passive implantable medical devices (IMDs, implants) are, for example, a pacemaker (with leads), an implantable cardiac monitor (ICM), an Implantable Leadless Pacer (ILP), an Implantable Leadless Pressure Sensor (ILPS), an Implantable Cardiac Defibrillator (ICD) or a Subcutaneously Implanted Cardiac Defibrillator (S-ICD), and a device that delivers spinal cord stimulation (SCS), deep brain stimulation (DBS) or neurostimulation. Such IMD may deliver one or more therapeutic substances, (e.g., a drug pump), may contain sensors that collect physiological signals to monitor the health status of the patient and/or deliver a therapy to the patient, for example using electromagnetic waves or electrical output. Such collected signals or therapy information may be transmitted as data to an external device (e.g., smartphone, computer, remote server or wand) using a communication unit, wherein the external device is an end station or a relay station which is located at least partly extracorporeally. The data collected from the various sensors of the IMD can include, but are not limited to, ECG, impedance, activity, posture, heart sounds, pressure, respiration data and other data.

Usually, such IMD comprises a processor for data processing and a communication unit configured to bi-directionally exchange signals with a communication unit of the external device. Accordingly, the communication unit of the external device is configured for bidirectional signal exchange with the communication unit of the IMD. The communication unit of the external device, for example, produces and sends signals to the transceiver module of the IMD in form of requests, for example for receiving data concerning the health status of the patient or IMD status from the IMD or for programming (e.g., to configure the IMD to apply appropriate therapies to the patient). Additionally, the external device may provide an electromagnetic field for wireless power transfer to charge a rechargeable battery of the IMD, for example using inductive or capacitive coupling. In some cases the communication unit of the IMD is operated in an active mode and in a sleep mode to reduce energy consumption. A pre-defined signal provided by the communication unit of the external device triggers switching of the communication unit of the IMD from the sleep mode into the active mode (so-called wake up).

As IMD sizes continue to shrink at a rate that outpaces the ability for battery capacity densities to improve, the use of low-power circuitry plays an increasingly central role in supporting product service time needs. Unfortunately, developing and incorporating low-power circuitry often involves making trade-offs where lower-frequency on-board clock operation and the reduction in power used to drive subsystem components cause them to run slower through compromised start-up and configuration management times. An area where this provides a direct potential consequence for a user (e.g., a patient or technician or a health care practitioner (HCP) such as a doctor or a nurse) with such products involves the establishment of a communication link by the external device. Such external device used for data exchange, for recharging of secondary cells, or otherwise have often included LED indicators to report when communication is functional but have not incorporated any sophistication to assess when the external device has made contact with the IMD. Moving the external device too quickly or in ways that sweep it out of a position where it has sent a wake-up cue to the IMD but is now out of range for receiving a response prolong the startup of viable information and/or power exchange with the IMD. Presently the external device offers feedback/reporting of whether or not communication is "good" (i.e., has a sufficient signal strength) with the underlying IMD but if any substantial delay in "bringing up" the IMD is in effect, and if the user is accustomed to moving the external device about in search of the IMD, the response from the IMD may occur after the external device has moved out of range to facilitate its receipt.

In document US 2020/0178802 A1 a system and a method for improving the security of an operation for writing the memory of an active implantable medical device by long distance telemetry, in particular via network connection with a writing main device, is described. The system and method comprise an intermediate proximity device allowing communication between the active implantable medical device and the main writing device and at least one unlocking tool allowing access to the active implantable medical device.

In document US 2020/405149 A1 a computer implemented method, system and device are described. The method transmits an energizing signal from an external antenna, coupled to a local external device (LED), to an implanted antenna of a passive implanted medical device (PIMD). The energizing signal is transmitted while the external antenna is at first and second positions. The method receives, at the external antenna, first and second energy transfer characteristic (ETC) values associated with the first and second positions, respectively. The method is under control of one or more processors configured with program instructions. The method analyzes the first and second ETC values to determine a difference therebetween. The method provides an energy transfer level (ETL) indicator based on the difference between the first and second ETC values. The ETL indicator provides feedback regarding a degree of energy transfer associated with at least one of the first and second positions.

In document US 6 510 345 B1 a method of and a system are described for preventing unwanted alternating current magnetic fields transmitted from an external source from disrupting electrical circuitry within an implantable medical device, while permitting desired communications between the implantable medical device and a programmer detected in proximity to the implantable medical device. The implantable medical device is capable of communication with at least one implantable lead position within a heart of a patient. The method includes activating an electrical device to create a short circuit in parallel with an inductive transreceiver coil of the implantable medical device, thereby shorting out the inductor transreceiver coil. In this mode, alternating current magnetic fields do not effect electrical circuitry within the implantable medical device. The method further includes detecting a direct current magnetic field indicating the presence of a programmer located in proximity to the implantable medical device. The electrical device shorting out the inductive transreceiver coil is deactivated and thereby opened during detection of the presence of the programmer in proximity to the implantable medical device such that the implantable medical device and the programmer can communicate via the inductive transreceiver coil.

In document US 2011/0184491 A1 wireless communication with an implantable medical device is described. An implantable medical device (IMD) may initiate the establishment of a communication session by sending a wakeup communication to an external device with which the IMD desires to communicate. The external device may operate in low power state (sometimes referred to as a sleep state or a low current state) in which the external device occasionally powers up telemetry circuitry to monitor for the wakeup communication from the IMD. Upon receiving the wakeup communication, the external device transitions from the low power state to a high power state to establish the communication session with the IMD. Operating the external device in the low power state when no communication session is established reduces the amount of power consumed by the external device when no telemetry is occurring, thus enabling the external device to be powered by a battery.

Accordingly, it is desirable to provide a system comprising an IMD and an external device as well as an external device and a respective operation method that enhances the quality of user interaction.

The above object is solved by an external device with the features of claim 1, by a respective operation method with the features of claim 7, by a computer program product with the features of claim 13, by a computer readable data carrier with the features of claim 14 and a system with the features of claim 15. The computer program product may be a software routine (e.g., related to hardware support means within the external device and/or the implantable medical device).

In particular, the above problem is solved by an external device configured to communicate with an implantable medical device implanted within a patient's body using a communication unit, wherein the external device further comprises a processor, at least one proximity sensor and at least one motion sensor, which is configured to detect whether the external device moves and to provide a respective motion signal, wherein the external device is movable with regard to the patient's body (e.g. by a user), to find and establish a communication connection of the communication unit and the implantable medical device, wherein the communication unit, the at least one proximity sensor and the at least one motion sensor are electrically connected to the processor, wherein the processor is configured to assess the current motion signal of the at least one motion sensor if a signal received from the at least one proximity sensor indicated that the external device is located near the patient's body and/or near the implantable medical device and to provide a movement guidance information (e.g. to the user or a module moving the external device) based on the assessment of the current motion signal of the at least one motion sensor.

The external device is always located at least partially extracorporeally. The external device may be a computer, a smartphone, a server or similar computing device comprising a communication unit and a processor. The external device may be a so-called patient remote device (PR) or wand-based device which utilizes a transceiver head for routing communication between the IMD and either a programmer or a remote monitoring server. The external device may give the user the ability to change the active therapy program of the IMD, control its stimulation signal amplitude, turn stimulation signal on/off, view battery status and/or provide an electromagnetic field for wireless power transfer to the IMD. The described functionality of the external device and below method may be realized in part by a corresponding app.

The implantable medical device is any device as indicated above which is configured to be implanted partly or fully within a patient's body and is configured to monitor the health condition of a patient and/or to deliver a therapy to the patient. Particularly, the medical device may be, for example, any implant, a pacemaker, ILP, ICD, Subcutaneously Implanted Cardiac Defibrillator (S-ICD), a device that delivers SCD or an Implantable Cardiac Monitor (ICM), an implantable drug delivery device, a cochlear implant or otherwise. The IMD is at least partly implantable within the patient's body. After implantation, the IMD provides communication with the external device using a communication unit.

The above object is also solved by a system comprising the above defined external device and an implantable medical device, wherein the external device (e.g., its communication unit) is configured to communicate with the IMD, for example with a respective communication unit of the IMD. The communication units are configured such that they use the same pre-defined communication method.

The data and/or power exchange between the communication unit of the IMD and the communication unit of the external device may be wireless in nature and comprise conducted acoustic, galvanic, and/or magnetically/inductively coupled formats where a wand-based hardware element or otherwise is necessarily in direct physical contact with the patient. Additionally, in some instances, such data and/or power exchange may occur (at least in part) over the air (i.e., without demanding direct physical contact with the patient by hardware elements of the external device) via electromagnetic waves, using means inclusive of, MedRadio/MICS/MEDS, EDGE, EV-DO, Flash-OFDM, GPRS, HSPA, LoRaWAN, RTT, UMTS, Narrowband IoT, Bluetooth, WLAN (WiFi), ZigBee, NFC, LTE, Wireless USB, Wibree (BLE), Ethernet and/or WiMAX radio frequency methods, and furthermore may additionally, or instead, leverage IrDA or free-space optical communication (FSO) tactics. Accordingly, the respective communication protocols, often with the same name (i.e., a system of rules that allows two or more entities of a communications system to transmit information via a kind of variation of a physical quantity, defining rules, syntax, semantics and synchronization of communication and possible error recovery methods and being implemented by hardware, software or a combination of both) or cooperating protocols (protocol suites) may be used, for example the TCP/IP suite (e.g. IPv6, TCP, UDP, SMTP, HTTP/2),TLS/SSL, IPX/SPX, X.25, AX.25, ebXML, Apple Talk, Bluetooth (e.g., BR/EDR), Bluetooth 4.0 (BLE), ZigBee, NFC, IEEE 802.11 and 802.16, company-proprietary methods, etc.. After implantation of the IMD, the communication path comprises a path section within the tissue of the patient's body. The communication unit of the IMD and the communication unit of the external device may be configured for bi-directional communication with each other and therefore each communication unit may comprise a transceiver for signals, that incorporates at least some form of antenna. The communication module of the IMD and the communication unit of the external device may comprise the respective hardware and software adapted to the used communication techniques / communication protocols.

Additionally, either the entire external device or a module (e.g., wand) associated with the external device is freely movable with respect to the patient's body in support of the establishment of a communication connection of the external device's communication unit with the communication unit of the IMD by means of user (e.g., HCP, patient, or technician) engagement. To establish the communication connection, the communication unit of the external device sends pre-defined signal(s)/message(s) with a pre-defined signal strength (amplitude), signal length and/or frequency. As soon as the communication unit of the IMD receives such signal(s), the communication sends a respective pre-defined confirmation signal(s)/message(s) to the communication unit of the external device. As soon as the communication unit of the external device receives such confirmation signal(s)/message(s) the processor of the external device recognizes that the communication connection between the IMD and the external device is established. The communication connection may then be used and maintained for data transmission or may provide or trigger wireless power transfer to the IMD.

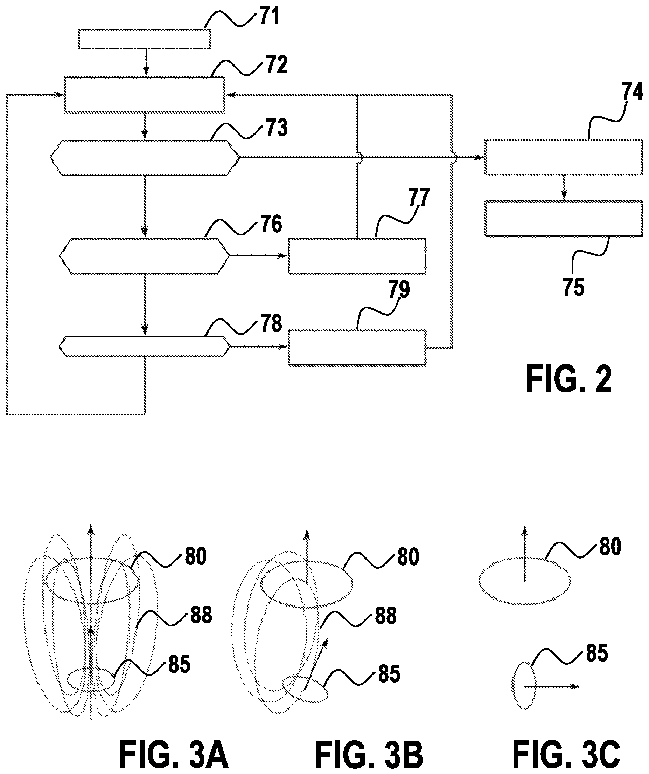

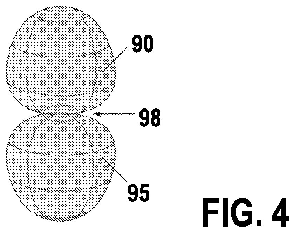

In one embodiment the communication employs inductive coil-based physical means. In this case, to provide reliable and robust communication, certain alignments between the coil(s) of the external device (see reference number 80 in Fig. 3a to c) and the coil (see reference number 85 in Fig. 3a to c) residing in the underlying IMD is provided - a best case being one where the two coils provide coincident (as shown in Fig. 3a) or antiparallel normal vectors (see arrows from the respective center point of each coil 80, 85). If the flux lines 88 associated with the IMD's coil 85 and external device's coil 80 can co-mingle and couple effectively, communication and/or power exchange across such a link proves feasible. On the other hand, if the normal vectors of the two coils associated with this link are perpendicular with respect to one another (as shown in Fig. 3c) no viable communication proves feasible. Fig. 3b shows the case in which coupling is less effective compared to Fig. 3a because the normal vectors are tilted. To further expand on the notion that either coincident (as shown in Fig. 3a) or antiparallel alignments are supported, Fig. 4 provides a representative communication and/or power exchange lobe map that supports viable coupling subject to the placement of an external device in the near field of a coil of an IMD - the external device's coil being oriented in a manner where its normal vector points either toward (coincident) or away from (antiparallel) the center of the IMD coil. Coupling is supported if the external device is positioned within either the "positive" or "negative" (naming of polarity is arbitrary) lobes 90, 95 but not if it resides within the "null" (see reference number 98 in Fig. 4). The differences in polarity change the phase of the communication and/or power exchange but do not deny intended interactions. Further, hopping or transitioning from one lobe to another in the middle of communication or power coupling still supports engagements between the IMD and the external device and therefore the key alignment that the above-defined external device aims to provide guidance in avoiding conditions where the wand is perpendicular to the implant coil's normal vector (Fig. 3c) (i.e., those that stations the external device coil within the null of the IMD coil 85 - again, see reference number 98 in Fig. 4).

For data/signal processing, the external device and the IMD each comprise or may comprise a dedicated processor which is generally regarded as a functional unit of the external device or the IMD, that interprets and executes instructions comprising an instruction control unit and an arithmetic and logic unit. The processor may comprise a microprocessor, a controller, a digital signal processor (DSP), an application specific integrated circuit (ASIC), a field-programmable gate array (FPGA), discrete logic circuitry or any combination thereof. Alternatively, or additionally, the processor may be realized using integrated dedicated hardware logic circuits, in particular, in the case of an IMD due to its compact size and affilate power limitatiosn. The processor may furthermore comprise a plurality of processing subunits.

The external device comprises at least one proximity sensor electrically connected to the processor, wherein the proximity sensor detects whether the external device is located near the patient's body and/or near the IMD, i.e. the proximity sensor detects whether the patient's body and/or the IMD is within a pre-defined spatial distance from the external device. In one embodiment, the at least one proximity sensor comprises an optical sensor and/or an inductive sensor and/or a sensor using radar, sonar and/or ultrasound. The proximity sensor may be an inductive metal detector that is configured to determine whether or not a metal object resides within the vicinity of a coil provided within the external device. This sensor may produce an alternating magnetic field that induces eddy currents into the housing of the underlying IMD, if it is present. The presence of such eddy currents, in turn alter the magnetic field incident from the external device which is monitored by the processor (e.g., using a magnetometer) so that the presence of the IMD may be detected. By adding at least one proximity sensor to the external device the external device is "self aware" of conditions where the external device has been placed in contact with the patient and/or the IMD. In one embodiment, the external device may provide information, (e.g. to the user), regarding whether it has detected proximity to the patient and/or IMD. In one embodiment, the proximity sensor enables the external device to recognize whether it has been placed on the patient's chest, back, or other body surface (though not necessarily distinguishing which). Depending upon the sophistication employed, such sensor feedback may include intelligence to understand whether or not the external device had made contact with anatomy or compliant surfaces as opposed to something non-physiologic such as setting the external device on the hard surface of a counter or desk by, for instance, seeking signatures specific to underlying bloodflow using oxygen saturation assessments; recognizing motion signals associated respiration operations (i.e., is the surface being touched "breathing" or not); monitoring for adjacent temperature profiles indicative of presence of a warm-blooded human (e.g., is the surface at nominally 37C or otherwise); or leveraging capacitive sensors akin to smartphone touch screens which can readily distinguish between engagements with human skin and otherwise subject to the contacting surface's ability to stack charge.

Further, the external device comprises at least one motion sensor (e.g. an accelerometer, a mercury switch, a tuned capacitor, an ultrasound, microwave, or infrared Doppler effect component, or otherwise) electrically connected to the processor. The acceleration sensor may detect an acceleration (i.e. the rate of change of velocity) related to a two-dimensional or three-dimensional movement of the external device over time in the external device's own instantaneous rest frame. The at least one acceleration sensor may detect both the magnitude and the direction of the acceleration, as a vector quantity, and may be used to sense orientation (because the direction of weight changes), coordinate acceleration and/or vibration. Examples of an acceleration sensor are piezoelectric accelerometer, laser accelerometer, pendulous integrating gyroscopic accelerometer, accelerometer using magnetic induction, optical accelerometer, strain gauge, accelerometer using surface acoustic waves and/or any combination of above principles. The at least one acceleration sensor may comprise micromachined microelectromechanical systems (MEMS) accelerometers to detect changes in the position of the external device.

The processor is configured to assess the current motion signal of the at least one motion sensor if a signal received from the at least one proximity sensor indicated that the external device is located in direct contact with the patient's body and/or near the IMD. Near proximity to the IMD would notionally include separation distances between the external device (or device wand) and the IMD of approximate 20 cm or less. Further, the processor provides a movement guidance information to the user, based on the assessment of the current motion signal of the at least one motion sensor. For example, if the motion value exceeds a pre-defined motion threshold value in at least one direction, the processor provides a movement guidance information to the user, for example causing a display on the external device to show information such as "please move external device more slowly". The external device thereby supports dynamic and active guidance to avoid having the user unwittingly compete with effective external device/IMD link coordination. The movement guidance information provided to the user gives the system increased opportunities for establishing a communication link, warning the user to not move the external device with excessive haste unless, by doing so, they wish to delay start-up procedures. In one embodiment, where the external device positioning was not successful (i.e., did not lead to establishment of a communication link between the external device and the IMD - for example, recognized by a time-out) the processor is configured to provide a positioning change recommendation to the user, instructing them to try a new position of the external device relative to the patient's body and/or the IMD.

The above-described external device minimizes user frustrations associated with efforts to establish a communication and/or power-exchange link with an underlying IMD by offering real-time messaging/feedback on the process. The related method is especially well-suited to support IMDs that can benefit from incorporating a slower on-board oscillator to manage start-up (i.e., uses lower power, is less expensive) enabling access to better implantable product service times, expanded supplier selection/redundancy in IMD module component selection, and improved sales margins. It is further especially well-suited for improving engagements with IMDs stationed deep within the patient anatomy such as leadless implants which cannot be guaranteed to present the same, known-optimal coil orientations common to devices optimized for subcutaneous implantation.

In one embodiment, the processor is configured to suspend assessing the current motion signal of the at least one motion sensor in the external device and/or to provide a communication confirmation information, e.g. to the user by means of the external device, after the communication unit within the external device has received a pre-defined response signal from the implantable medical device indicating that a communication connection link has been established.

As indicated above, in one embodiment, the processor is configured to provide a position change information, e.g. to the user, requesting the user to change the position after a first pre-defined time period elapsed after starting the process of finding the communication connection and/or after a second pre-defined time period elapsed after a signal received from the at least one proximity sensor indicated that the external device is located near the patient's body and/or near the implantable medical device.

In one embodiment, the movement guidance information and/or the communication confirmation information and/or the position change information is an information that is haptically and/or optically and/or audibly perceptible by the user. Accordingly, the external device may comprise a respective display and/or a speaker and/or a vibration unit configured to provide an information that the user feels haptically. The display and/or speaker and/or vibration unit is electrically connected to and controlled by the processor such that the respective information is provided to the user.

In one embodiment, the processor is configured to trigger transition of the communication unit from a sleep mode into an active mode if a signal received from the at least one proximity sensor indicated that the external device is located on the patient's body and/or near the implantable medical device. In the sleep mode the communication unit of the external device has a very low power consumption. In the sleep mode, establishment of a communication connection to the communication unit of the IMD is not enabled. Only in the active mode, the communication unit of the external device may establish and maintain a communication connection to the communication unit of the IMD.

In one embodiment each of the IMD and the external device may comprise a memory unit that may include any volatile, non-volatile, magnetic, or electrical media, such as a random access memory (RAM), read-only memory (ROM), non-volatile RAM (NVRAM), electrically-erasable programmable ROM (EEPROM), flash memory, or any other memory device.

The IMD may comprise further modules such as a power supply such as a battery, at least one sensor for retrieving physiological signals from the patient and/or a signal generator for generating, for example, electrical or electromagnetically therapy signals in order to provide the therapy to the patient. The transceiver module, the memory module, the power supply, the at least one sensor and/or the signal generator may be electrically connected to the processor. The components of the IMD may be located within a hermetically sealed housing.

The above problem is further solved, for example, by an operation method of an external device configured to communicate with an implantable medical device implanted within a patient's body using a communication unit, wherein the external device further comprises a processor, at least one proximity sensor and at least one motion sensor, which detects whether the external device moves and provides a respective motion signal, wherein the external device (or a critical sub-module of it that can be placed in direct contact with the patient) is movable with reference to the patient's body, e.g. by a user, to find and establish a communication connection of the communication unit and the implantable medical device, wherein the communication unit, the at least one proximity sensor and the at least one motion sensor are electrically connected to the processor, wherein the processor assesses the current motion signal of the at least one motion sensor if a signal received from the at least one proximity sensor indicated that the external device is located near the patient's body and/or near the implantable medical device and provides a movement guidance information, to the user, based on the assessment of the current motion signal of the at least one motion sensor.

In more detail, the method comprises the following steps, wherein many features of the method are already explained with regard to the external device, the IMD and the system above. It is therefore referred to the above explanation also with regard to the operation method.

In one embodiment of the method, the processor suspends assessing the current motion signal of the at least one motion sensor and/or provides communication confirmation information to the user, after the communication unit receives a pre-defined response signal from the implantable medical device indicating that a communication connection with the implantable medical device was established.

In one embodiment of the method, the processor provides a position change information to the user, requesting the user to change the position after a first pre-defined time period elapsed after starting the process of finding the communication connection and/or after a second pre-defined time period elapsed after a signal received from the at least one proximity sensor indicated that the external device is located near the patient's body and/or near the implantable medical device.

In one embodiment of the method, the movement guidance information and/or the communication confirmation information and/or the position change information is an information that is haptically and/or optically and/or audibly perceptible by the user.

In one embodiment of the method, the at least one proximity sensor detects optical signals and/or an electromagnetic signals and/or radar, sonar and/or ultrasound signals.

In one embodiment of the method, the processor triggers transition of the communication unit from a sleep mode into an active mode if a signal received from the at least one proximity sensor indicated that the external device is located near the patient's body and/or near the implantable medical device.

The above method may, further, be realized as a computer program which comprises instructions which, when executed, cause the system (e.g. the external device and/or the IMD) to perform the steps of the above operation method which is a combination of above and below specified computer instructions and data definitions that enable computer hardware to perform computational or control functions or which is a syntactic unit that conforms to the rules of a particular programming language and that is composed of declarations and statements or instructions needed for a above and below specified function, task, or problem solution.

Furthermore, the above defined method may be part of a computer program product comprising instructions which, when executed by a processor, cause the processor to perform the steps of the above defined method. Accordingly, a computer readable data carrier storing such computer program product is disclosed.

The present invention is defined by the appended claims and will now be described in further detail with reference to the accompanying schematic drawing, wherein
- Fig. 1: shows an embodiment of the system comprising an IMD and an external device,
- Fig. 2: depicts a flow diagram of an operation method of the external device,
- Figs. 3A-3C: depict examples of alignment of coils of the communication units of IMD and of the external device, wherein in Fig. 3A the axes of the coils are parallel, in Fig. 3B the axes of the coils are tilted and in Fig. 3C perpendicular to each other, and
- Fig. 4: shows a representative mapping of the regions of a coil of the communication unit of the IMD in space (i.e. lobes) where communication and/or power transmission may be supported using the coil of the communication unit of the external device.

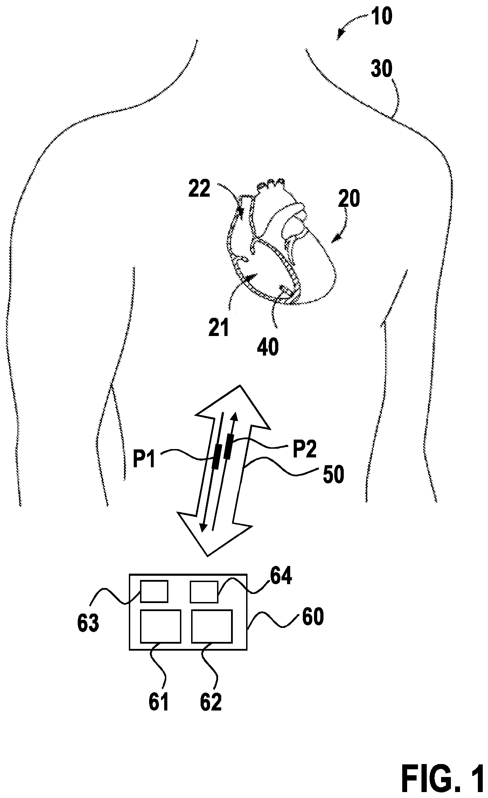

Fig. 1 shows an example system 10 and a heart 20 (with right ventricle 21 and right atrium 22) of a patient 30. The system 10 comprises a leadless ventricular pacemaker device 40 (hereinafter "ILP 40") as an example for an IMD and an external device in form of a wand-based embodiment 60. ILP 40 may be configured to be implanted within the right ventricle 21 of the heart (as shown in Fig. 1) and to pace this ventricle, sense intrinsic ventricular depolarizations and impedance, and inhibit ventricular pacing in response to detected ventricular depolarization. The ILP 40 further comprises a communication unit with a transceiver for sending to and receiving signals from a communication unit 62 of the external device 60. The wand 60 comprises a processor 61, the communication unit 62 containing a transceiver chip for bidirectional communication with the communication unit of the ILP 40, a motion sensor in form of an accelerometer 63 to detect whether the wand 60 moves as could be assessed by means of the determination of DC or AC RMS amplitudes crossing thresholds set to varying levels that have been clinically verified/validated as ones indicative of critical motion dynamics relevant for generating user feedback. The wand 60 further comprises a proximity sensor 64 based, for example, on ultrasound or optical signals. The ILP 40 comprises modules such as a processor, a data memory module, a signal generator unit for providing treatment signals (e.g., pacing signals), a measurement unit comprising an ECG measuring unit and a DC impedance sensor. Further, it comprises a power source wherein the modules are electrically connected to each other. The power source may include a battery (e.g., a rechargeable or non-rechargeable battery). The data memory module may include any memory type mentioned above.

The wand 60 is a portable external device sub-system which can be moved in relation to the patient's 30 body and/or the ILP 40. The communication unit 62 is configured to exchange messages/signals in the form of packets with the communication unit of the ILP 40, wherein the processor 61 and the communication unit 62 are electrically connected with each other. Further, the accelerometer 63 and the proximity sensor 64 are electrically connected with the processor 61, as well. The bi-directional exchange of messages/signals P1, P2 with the ILP 40 associated with an established communication connection, is symbolized by the double arrow 50 in Fig. 1. The wireless communication of the external device 60 with the ILP 40 may be facilitated, for example, by acoustic, conducted, galvanic and/or mechanically/inductively coupled methods (i.e., those that demand direct wand application to the patient as RF-based methods are unable to access the deep implanted leadless pacers). As such, the communication unit 62 of the wand 60 and the communication unit of the ILP 40 each comprises a suitable transceiver chip.

Prior to establishing the above-mentioned communication connection between the wand 60 and the ILP 40, the communication unit of the ILP 40 likely resides in a sleep mode utilizing only a very small energy amount. It may further prove possible for the communication unit 62 of the wand 60 to reside in sleep conditions in baseline states prior to the establishment of a communication link with the IMD and such conditions may potentially leverage signaling from the in-wand proximity and/or motion sensor as inputs to transitioning the wand away from such sleep conditions. In cases where the external device exits any such sleep conditions, it would begin efforts to establish a communication link and only subject to the receipt of affiliate messages from the external wand would the IMD, in turn, have a means for exiting its sleep state. If a user (e.g. physician) intends to use the wand 60 for communication with the implanted ILP 40, for example, during a follow-up to receive interrogate or program the ILP 40, the user moves the wand 60 to the patient's 30 chest. In this first step, the proximity sensor 64 is activated and detects, whether the wand 60 is in contact with the patient's chest (see step 71 in Fig. 2) or near enough to the IMD itself.

If the proximity sensor 64 detects that the wand 60 is close to the ILP 40 or the chest region of the patient 30, the operation continues with step 72 in which the communication unit 62 of the wand 60 is activated and an in-process-timer is started in step 72. In step 72 the communication unit 62 of wand 60 begins sending activation signals to the communication unit of the ILP 40 to establish a communication connection. The process is further visualized to the user by an auditory, visible, and/or haptic information indicative of "in process" conditions.

In step 73 the processor 61 and the communication unit 62 of the external device assess the signals received by the communication unit 62 with regard to whether these signals contain any response signal of the communication unit of the ILP 40 indicating successful establishment of a communication connection between the wand 60 and the ILP 40. The reporting to the system and/or user is controlled by the processor 61.

If a pre-defined response signal indicating establishment of such communication connection is received, the process continues with step 74. In this step the wand 60 provides the auditory, visible, and/or haptic information to the user that the communication connection was established, for example by showing "engaged" on a possible relevant display. In one embodiment, the message "engaged" is shown on the display as long as the communication connection with the ILP 40 is established.

In the next step 75 the user feedback on the wand 60 provides information regarding the signal strength of the communication signal received from the ILP 40. For that, the wand 60 likely, in preferred embodiments, uses a signal strength indicator illuminated in red if the signal strength is low, in yellow if the signal strength is medium and in green if the signal strength is good. Beginning from step 75 the "normal" communication between the ILP 40 and the wand 60 may take place, for example to send ECG and therapy data from the ILP 40 to the wand 60. The communication is disconnected after all requested data are received by the wand 60 or after a second timer time-out occurred. The communication units, the proximity sensor and the motion sensor then return into their sleep mode.

If the processor 61 does not recognize any confirmation by the pre-defined response signal that a communication connection between the wand 60 and the ILP 40 was established, the process continues with step 76. In this step the processor 61 assesses signals received from the motion sensor (e.g., accelerometer) 63 which was activated together with the communication unit 62 in step 72. If the accelerometer signal indicates that the wand 60 is fast moved with regard to the patient, the process continues with step 77 in which the external device reports a movement guidance information to the user, for example "stop moving wand" or "move the wand more slowly" to encourage the user to engage with the wand 60 in preferential ways. Then, the process continues with step 72.

If the accelerometer signal indicates in step 76 that the wand is only slowly moved or not moved, the process continues with step 78 in which the processor 61 queries the value of the in-process timer which was started in step 72 (or alternatively, and perhaps preferentially, an in-process timer that was started upon entry to step 78). If the in-process-timer has already reaches its pre-defined maximum value, the process continues with step 79 instructing the user with new movement guidance information suggesting they "try a new wand position" to improve the likelihood of establishing the communication connection. Then, the process continues with step 72. The process also continues with the step 72 if the in-process-timer has not reached its maximum value, yet.

Within the flow diagram of Fig. 2 general terms have been deliberately used as the format of alerts and displays could take a variety of forms. Light-emitting diodes, auditory beeps, and vibrational haptic responses are but a few explicit examples that could serve the intent of this dynamic feedback support, in particular in steps 72, 74, 75, 77, 79. The basic format of these wand interactions centers on having users "give the wand a chance" by discouraging the user from rapidly hunting for an underlying ILP 40 through the use of swift wand motions. By throttling such rapid movements, those trying to establish a communication or power-exchange link with the implant are substantially less apt to reposition the wand 60 prior to giving the ILP 40 sufficient time to recognize the activation signal and respond thereby enabling better, cleaner, faster coordination between the wand 60 and the ILP 40. Conversely, in cases where the wand has sat idle for long durations while in contact with the patient but hasn't established a communication link, the system encourages the user to try new wand positioning as continued residence in the static state is not likely to result in the establishment of a link as an IMD is likely not located within range of the wand.

Based on the flow diagram of Fig. 2 a display and/or alert method is provided which may use LEDs, auditory cues, haptic feedback, or a variety of other physical means to inform the user of the status of and the recommended engagements that should be made with the wand to hasten startup sequences. Further, an algorithm for taking the sensor inputs and, in turn, managing the displays/alerts/information for the user in accordance with the basic notion offered in Fig. 2 above is described.

By leaning on the use of built-in sensor capabilities taking the form of at least one motion sensor 63 and a proximity sensor 64 the wand 60 may be "self aware" as to when it has been placed in contact with a patient's chest (to initiate communication) as well as how spatially stable (i.e., moving with excessive haste, moving slowly, or not moving at all) the wand 60 is following such placements. This information may then be sent to a user feedback mechanism (preferentially one or more LEDs on the wand) to inform the user on how they best engage - especially throttling their inclination to quickly move the wand 60 about in search of the ILP which may unwittingly further extend startup durations.

## Claims

1. An external device (60) configured to communicate with an implantable medical device (40) implanted within a patient's (30) body using a communication unit (62), wherein the external device (60) further comprises a processor (61), at least one proximity sensor (64) and at least one motion sensor (63), which is configured to detect whether the external device moves and to provide a respective motion signal, wherein the external device (60) is movable with regard to the patient's body to find and establish a communication connection (50) of the communication unit and the implantable medical device, wherein the communication unit (62), the at least one proximity sensor (64) and the at least one motion sensor (63) are electrically connected to the processor (61), wherein the processor (61) is configured to assess the current motion signal of the at least one motion sensor if a signal received from the at least one proximity sensor (64) indicated that the external device (60) is located near the patient's body and/or near the implantable medical device (40) and to provide a movement guidance information based on the assessment of the current motion signal of the at least one motion sensor.

2. The external device of claim 1, wherein the processor (61) is configured to suspend assessing the current motion signal of the at least one motion sensor (63) and/or to provide a communication confirmation information to the user after the communication unit (62) received a pre-defined response signal from the implantable medical device (40) indicating that a communication connection with the implantable medical device (40) was established.

3. The external device of any of the previous claims, wherein the processor (61) is configured to provide a position change information to the user requesting the user to change the position after a first pre-defined time period elapsed after starting the process of finding the communication connection (50) and/or after a second pre-defined time period elapsed after a signal received from the at least one proximity sensor (64) indicated that the external device (60) is located near the patient's body and/or near the implantable medical device (40).

4. The external device of any of the previous claims, wherein the movement guidance information and/or the communication confirmation information and/or the position change information is an information that is haptically and/or optically and/or audibly perceptible by the user.

5. The external device of any of the previous claims, wherein the at least one proximity sensor (64) comprises an optical sensor and/or an inductive sensor and/or a sensor using radar, sonar and/or ultrasound.

6. The external device of any of the previous claims, wherein the processor (61) is configured to trigger transition of the communication unit (62) from a sleep mode into an active mode if a signal received from the at least one proximity sensor indicated that the external device is located near the patient's body and/or near the implantable medical device.

7. An operation method of an external device (60) configured to communicate with an implantable medical device (40) implanted within a patient's (30) body using a communication unit (62), wherein the external device further comprises a processor (61), at least one proximity sensor (64) and at least one motion sensor (63), which detects whether the external device moves and provides a respective motion signal, wherein the external device (60) is movable with regard to the patient's body to find and establish a communication connection (50) of the communication unit (62) and the implantable medical device (40), wherein the communication unit (62), the at least one proximity sensor (64) and the at least one motion sensor (63) are electrically connected to the processor (61), wherein the processor (61) assesses the current motion signal of the at least one motion sensor if a signal received from the at least one proximity sensor (64) indicated that the external device (60) is located near the patient's (30) body and/or near the implantable medical device (40) and provides a movement guidance information based on the assessment of the current motion signal of the at least one motion sensor (64).

8. The method of claim 7, wherein the processor (61) suspends assessing the current motion signal of the at least one motion sensor (64) and/or provides a communication confirmation information after the communication unit (62) received a pre-defined response signal from the implantable medical device indicating that a communication connection (50) with the implantable medical device was established.

9. The method of any one of the claims 7 to 8, wherein the processor (61) provides a position change information requesting to change the position after a first pre-defined time period elapsed after starting the process of finding the communication connection and/or after a second pre-defined time period elapsed after a signal received from the at least one proximity sensor indicated that the external device is located near the patient's body and/or near the implantable medical device (40).

10. The method of any one of the claims 7 to 9, wherein the movement guidance information and/or the communication confirmation information and/or the position change information is an information that is haptically and/or optically and/or audibly perceptible by a user.

11. The method of any one of the claims 7 to 10, wherein the at least one proximity sensor (64) detects optical signals and/or electromagnetic signals and/or radar, sonar and/or ultrasound signals.

12. The method of any one of the claims 7 to 11, wherein the processor (61) triggers transition of the communication unit (62) from a sleep mode into an active mode if a signal received from the at least one proximity sensor indicated that the external device (60) is located near the patient's body and/or near the implantable medical device (40).

13. A computer program product comprising instructions which, when executed by the processor of the external device according to any of claims 1 to 6, cause the processor (61) to perform the steps of the method according to any of the claims 7 to 12.

14. Computer readable data carrier storing a computer program product according to claim 13.

15. System comprising an implantable medical device (40) and the external device (60) of any of the claims 1 to 6.

## Patentansprüche

1. Externes Gerät (60), das so konfiguriert ist, dass es mit einem implantierbaren medizinischen Gerät (40), das in den Körper eines Patienten (30) implantiert ist, über eine Kommunikationseinheit (62) kommuniziert, wobei das externe Gerät (60) ferner einen Prozessor (61), mindestens einen Näherungssensor (64) und mindestens einen Bewegungssensor (63) umfasst, der so konfiguriert ist, dass er erkennt, ob sich das externe Gerät bewegt, und ein entsprechendes Bewegungssignal liefert, wobei das externe Gerät (60) in Bezug auf den Körper des Patienten beweglich ist, um eine Kommunikationsverbindung (50) zwischen der Kommunikationseinheit und dem implantierbaren medizinischen Gerät zu finden und herzustellen, wobei die Kommunikationseinheit (62), der mindestens eine Näherungssensor (64) und der mindestens eine Bewegungssensor (63) elektrisch mit dem Prozessor (61) verbunden sind, wobei der Prozessor (61) so konfiguriert ist, dass er das aktuelle Bewegungssignal des mindestens einen Bewegungssensors auswertet, wenn ein von dem mindestens einen Näherungssensor (64) empfangenes Signal anzeigt, dass sich das externe Gerät (60) in der Nähe des Körpers des Patienten und/oder in der Nähe des implantierbaren medizinischen Geräts (40) befindet, und dass er auf der Grundlage der Auswertung des aktuellen Bewegungssignals des mindestens einen Bewegungssensors eine Bewegungsführungsinformation bereitstellt.

2. Externes Gerät nach Anspruch 1, wobei der Prozessor (61) so konfiguriert ist, dass er die Bewertung des aktuellen Bewegungssignals des mindestens einen Bewegungssensors (63) aussetzt und/oder dem Benutzer eine Kommunikationsbestätigungsinformation bereitstellt, nachdem die Kommunikationseinheit (62) ein vordefiniertes Antwortsignal von dem implantierbaren medizinischen Gerät (40) empfangen hat, das anzeigt, dass eine Kommunikationsverbindung mit dem implantierbaren medizinischen Gerät (40) hergestellt wurde.

3. Externes Gerät nach einem der vorhergehenden Ansprüche, wobei der Prozessor (61) so konfiguriert ist, dass er dem Benutzer eine Positionsänderungsinformation bereitstellt, die den Benutzer auffordert, die Position zu ändern, nachdem eine erste vordefinierte Zeitspanne nach dem Start des Prozesses zum Finden der Kommunikationsverbindung (50) verstrichen ist und/oder nachdem eine zweite vordefinierte Zeitspanne nach dem Empfang eines Signals von dem mindestens einen Näherungssensor (64) empfangen wurde, das anzeigt, dass sich das externe Gerät (60) in der Nähe des Körpers des Patienten und/oder in der Nähe des implantierbaren medizinischen Geräts (40) befindet.

4. Externes Gerät nach einem der vorherigen Ansprüche, wobei die Bewegungsführungsinformation und/oder die Kommunikationsbestätigungsinformation und/oder die Positionsänderungsinformation eine Information ist, die für den Benutzer haptisch und/oder optisch und/oder akustisch wahrnehmbar ist.

5. Externes Gerät nach einem der vorhergehenden Ansprüche, wobei der mindestens eine Näherungssensor (64) einen optischen Sensor und/oder einen induktiven Sensor und/oder einen Sensor umfasst, der Radar, Sonar und/oder Ultraschall verwendet.

6. Externes Gerät nach einem der vorhergehenden Ansprüche, wobei der Prozessor (61) so konfiguriert ist, dass er den Übergang der Kommunikationseinheit (62) von einem Schlafmodus in einen aktiven Modus auslöst, wenn ein von dem mindestens einen Näherungssensor empfangenes Signal anzeigt, dass sich das externe Gerät in der Nähe des Körpers des Patienten und/oder in der Nähe des implantierbaren medizinischen Geräts befindet.

7. Verfahren zum Betreiben eines externen Geräts (60), das so konfiguriert ist, dass es mit einem implantierbaren medizinischen Gerät (40), das in den Körper eines Patienten (30) implantiert ist, über eine Kommunikationseinheit (62) kommuniziert, wobei das externe Gerät ferner einen Prozessor (61), mindestens einen Näherungssensor (64) und mindestens einen Bewegungssensor (63) umfasst, der erkennt, ob sich das externe Gerät bewegt, und ein entsprechendes Bewegungssignal liefert, wobei das externe Gerät (60) in Bezug auf den Körper des Patienten beweglich ist, um eine Kommunikationsverbindung (50) zwischen der Kommunikationseinheit (62) und dem implantierbaren medizinischen Gerät (40) zu finden und herzustellen, wobei die Kommunikationseinheit (62), der mindestens eine Näherungssensor (64) und der mindestens eine Bewegungssensor (63) elektrisch mit dem Prozessor (61) verbunden sind, wobei der Prozessor (61) das aktuelle Bewegungssignal des mindestens einen Bewegungssensors auswertet, wenn ein von dem mindestens einen Näherungssensor (64) empfangenes Signal anzeigt, dass sich das externe Gerät (60) in der Nähe des Körpers des Patienten (30) und/oder in der Nähe des implantierbaren medizinischen Geräts (40) befindet, und auf der Grundlage der Auswertung des aktuellen Bewegungssignals des mindestens einen Bewegungssensors (64) eine Bewegungsführungsinformation bereitstellt.

8. Verfahren nach Anspruch 7, wobei der Prozessor (61) die Bewertung des aktuellen Bewegungssignals des mindestens einen Bewegungssensors (64) aussetzt und/oder eine Kommunikationsbestätigungsinformation bereitstellt, nachdem die Kommunikationseinheit (62) ein vordefiniertes Antwortsignal von dem implantierbaren medizinischen Gerät empfangen hat, das anzeigt, dass eine Kommunikationsverbindung (50) mit dem implantierbaren medizinischen Gerät hergestellt wurde.

9. Verfahren nach einem der Ansprüche 7 bis 8, wobei der Prozessor (61) eine Positionsänderungsinformation bereitstellt, die eine Änderung der Position anfordert, nachdem eine erste vordefinierte Zeitspanne nach dem Start des Prozesses zum Auffinden der Kommunikationsverbindung und/oder nach einer zweiten vordefinierten Zeitspanne nach dem Empfang eines Signals von dem mindestens einen Näherungssensor verstrichen ist, das anzeigt, dass sich das externe Gerät in der Nähe des Körpers des Patienten und/oder in der Nähe des implantierbaren medizinischen Geräts (40) befindet.

10. Verfahren nach einem der Ansprüche 7 bis 9, wobei die Bewegungsführungsinformation und/oder die Kommunikationsbestätigungsinformation und/oder die Positionsänderungsinformation eine Information ist, die für einen Benutzer haptisch und/oder optisch und/oder akustisch wahrnehmbar ist.

11. Verfahren nach einem der Ansprüche 7 bis 10, wobei der mindestens eine Näherungssensor (64) optische Signale und/oder elektromagnetische Signale und/oder Radar-, Sonar- und/oder Ultraschallsignale erfasst.

12. Verfahren nach einem der Ansprüche 7 bis 11, wobei der Prozessor (61) den Übergang der Kommunikationseinheit (62) von einem Schlafmodus in einen aktiven Modus auslöst, wenn ein von dem mindestens einen Näherungssensor empfangenes Signal anzeigt, dass sich das externe Gerät (60) in der Nähe des Körpers des Patienten und/oder in der Nähe des implantierbaren medizinischen Geräts (40) befindet.

13. Ein Computerprogrammprodukt, das Anweisungen umfasst, die, wenn sie vom Prozessor des externen Geräts gemäß einem der Ansprüche 1 bis 6 ausgeführt werden, den Prozessor (61) veranlassen, die Schritte des Verfahrens gemäß einem der Ansprüche 7 bis 12 auszuführen.

14. Computerlesbarer Datenträger, auf dem ein Computerprogrammprodukt gemäß Anspruch 13 gespeichert ist.

15. System, das ein implantierbares medizinisches Gerät (40) und das externe Gerät (60) gemäß einem der Ansprüche 1 bis 6 umfasst.

## Revendications

1. Dispositif externe (60) configuré pour communiquer avec un dispositif médical implantable (40) implanté au sein du corps d'un patient (30) en utilisant une unité de communication (62), dans lequel le dispositif externe (60) comprend en outre un processeur (61), au moins un capteur de proximité (64) et au moins un capteur de mouvement (63), qui est configuré pour détecter si le dispositif externe se déplace et pour fournir un signal de mouvement respectif, dans lequel le dispositif externe (60) est mobile par rapport au corps du patient pour trouver et établir une connexion en communication (50) de l'unité de communication et du dispositif médical implantable, dans lequel l'unité de communication (62), l'au moins un capteur de proximité (64) et l'au moins un capteur de mouvement (63) sont électriquement connectés au processeur (61), dans lequel le processeur (61) est configuré pour évaluer le signal de mouvement actuel de l'au moins un capteur de mouvement si un signal reçu de la part de l'au moins un capteur de proximité (64) a indiqué que le dispositif externe (60) est situé à proximité du corps du patient et/ou à proximité du dispositif médical implantable (40) et pour fournir une information de conseil de mouvement basée sur l'évaluation du signal de mouvement actuel de l'au moins un capteur de mouvement.

2. Dispositif externe selon la revendication 1, dans lequel le processeur (61) est configuré pour suspendre l'évaluation du signal de mouvement actuel de l'au moins un capteur de mouvement (63) et/ou pour fournir une information de confirmation de communication à l'utilisateur après que l'unité de communication (62) a reçu un signal de réponse prédéfini de la part du dispositif médical implantable (40) indiquant qu'une connexion en communication avec le dispositif médical implantable (40) a été établie.

3. Dispositif externe selon l'une quelconque des revendications précédentes, dans lequel le processeur (61) est configuré pour fournir une information de changement de position à l'utilisateur demandant à l'utilisateur de changer la position après une première période prédéfinie écoulée après le démarrage du processus consistant à trouver la connexion en communication (50) et/ou après une seconde période prédéfinie écoulée après qu'un signal reçu de la part de l'au moins un capteur de proximité (64) a indiqué que le dispositif externe (60) est situé à proximité du corps du patient et/ou à proximité du dispositif médical implantable (40).

4. Dispositif externe selon l'une quelconque des revendications précédentes, dans lequel l'information de conseil de mouvement et/ou l'information de confirmation de communication et/ou l'information de changement de position est une information qui est perceptible de manière haptique et/ou optique et/ou audible par l'utilisateur.

5. Dispositif externe selon l'une quelconque des revendications précédentes, dans lequel l'au moins un capteur de proximité (64) comprend un capteur optique et/ou un capteur inductif et/ou un capteur utilisant un radar, un sonar et/ou des ultrasons.

6. Dispositif externe selon l'une quelconque des revendications précédentes, dans lequel le processeur (61) est configuré pour déclencher la transition de l'unité de communication (62) d'un mode de veille à un mode actif si un signal reçu de la part de l'au moins un capteur de proximité a indiqué que le dispositif externe est situé à proximité du corps du patient et/ou à proximité du dispositif médical implantable.

7. Procédé de fonctionnement d'un dispositif externe (60) configuré pour communiquer avec un dispositif médical implantable (40) implanté au sein du corps d'un patient (30) en utilisant une unité de communication (62), dans lequel le dispositif externe comprend en outre un processeur (61), au moins un capteur de proximité (64) et au moins un capteur de mouvement (63), qui détecte si le dispositif externe se déplace et fournit un signal de mouvement respectif, dans lequel le dispositif externe (60) est mobile par rapport au corps du patient pour trouver et établir une connexion en communication (50) de l'unité de communication (62) et du dispositif médical implantable (40), dans lequel l'unité de communication (62), l'au moins un capteur de proximité (64) et l'au moins un capteur de mouvement (63) sont électriquement connectés au processeur (61), dans lequel le processeur (61) évalue le signal de mouvement actuel de l'au moins un capteur de mouvement si un signal reçu de la part de l'au moins un capteur de proximité (64) a indiqué que le dispositif externe (60) est situé à proximité du corps du patient (30) et/ou à proximité du dispositif médical implantable (40) et fournit une information de conseil de mouvement basée sur l'évaluation du signal de mouvement actuel de l'au moins un capteur de mouvement (64).

8. Procédé selon la revendication 7, dans lequel le processeur (61) suspend l'évaluation du signal de mouvement actuel de l'au moins un capteur de mouvement (64) et/ou fournit une information de confirmation de communication après que l'unité de communication (62) a reçu un signal de réponse prédéfini de la part du dispositif médical implantable indiquant qu'une connexion en communication (50) avec le dispositif médical implantable a été établie.

9. Procédé selon l'une quelconque des revendications 7 à 8, dans lequel le processeur (61) fournit une information de changement de position demandant de changer la position après une première période prédéfinie écoulée après le démarrage du processus consistant à trouver la connexion en communication et/ou après une seconde période prédéfinie écoulée après qu'un signal reçu de la part de l'au moins un capteur de proximité a indiqué que le dispositif externe est situé à proximité du corps du patient et/ou à proximité du dispositif médical implantable (40).

10. Procédé selon l'une quelconque des revendications 7 à 9, dans lequel l'information de conseil de mouvement et/ou l'information de confirmation de communication et/ou l'information de changement de position est une information qui est perceptible de manière haptique et/ou optique et/ou audible par un utilisateur.

11. Procédé selon l'une quelconque des revendications 7 à 10, dans lequel l'au moins un capteur de proximité (64) détecte des signaux optiques et/ou des signaux électromagnétiques et/ou des signaux radar, sonar et/ou ultrasonores.

12. Procédé selon l'une quelconque des revendications 7 à 11, dans lequel le processeur (61) déclenche la transition de l'unité de communication (62) d'un mode de veille à un mode actif si un signal reçu de la part de l'au moins un capteur de proximité a indiqué que le dispositif externe (60) est situé à proximité du corps du patient et/ou à proximité du dispositif médical implantable (40).

13. Produit de programme informatique comprenant des instructions qui, lorsqu'elles sont exécutées par le processeur du dispositif externe selon l'une quelconque des revendications 1 à 6, amènent le processeur (61) à réaliser les étapes du procédé selon l'une quelconque des revendications 7 à 12.

14. Support de données lisible par ordinateur stockant un produit de programme informatique selon la revendication 13.

15. Système comprenant un dispositif médical implantable (40) et le dispositif externe (60) selon l'une quelconque des revendications 1 à 6.
